# EUROPEAN PATENT APPLICATION

(11) **EP 3 878 980 A1**
(43) Date of publication of application: **15.09.2021**
(21) Application number: 20162860.9
(22) Date of filing: 12.03.2020
(51) Int. Cl.: C12R 1/07, A23K 10/18, C12N 1/20

(54) **AN ISOLATED BACILLUS ALTITUDINIS STRAIN AND ITS USE AS A PROBIOTIC**

(71) Applicant: Waterford Institute Of Technology, Waterford (IE); Agriculture and Food Development Authority (TEAGASC), Co. Carlow (IE)
(72) Inventor: Gardiner, Gillian, Fermoy, Co. Cork, (IE); Lawlor, Peadar, Fermoy, Co. Cork, (IE); Prieto, Maria, Waterford (IE); O'Sullivan, Laurie, Waterford (IE)
(74) Representative: Purdylucey Intellectual Property

(57) **Abstract**

An isolated *Bacillus altitudinis* strain as deposited with the National Collection of Industrial and Marine Bacteria under the Accession No. NCIMB 43558 on 27/01/202.

SEQUENCE LISTING

<110>  Waterford Institute of Technology
       Agriculture and Food Development Authority (Teagasc)

<120>  An isolated Bacillus altitudinis strain and its use as a
       probiotic

<130>  P13060EP00

<160>  2

<170>  PatentIn version 3.5

<210>  1
<211>  1417
<212>  DNA
<213>  Artificial Sequence

<220>
<223>  16S rRNA gene sequence amplicon

<400>  1
agtcgagcgg acagaaggga gcttgctccc ggatgttagc ggcggacggg tgagtaacac      60

gtgggtaacc tgcctgtaag actgggataa ctccgggaaa ccggagctaa taccggatag     120

ttccttgaac cgcatggttc aaggatgaaa gacggtttcg gctgtcactt acagatggac     180

ccgcggcgca ttagctagtt ggtgaggtaa cggctcacca aggcgacgat gcgtagccga     240

cctgagaggg tgatcggcca cactgggact gagacacggc ccagactcct acgggaggca     300

gcagtaggga atcttccgca atggacgaaa gtctgacgga gcaacgccgc gtgagtgatg     360

aaggttttcg gatcgtaaag ctctgttgtt agggaagaac aagtgcaaga gtaactgctt     420

gcaccttgac ggtacctaac cagaaagcca cggctaacta cgtgccagca gccgcggtaa     480

tacgtaggtg gcaagcgttg tccggaatta ttgggcgtaa agggctcgca ggcggtttct     540

taagtctgat gtgaaagccc ccggctcaac cggggagggt cattggaaac tgggaaactt     600

gagtgcagaa gaggagagtg gaattccacg tgtagcggtg aaatgcgtag agatgtggag     660

gaacaccagt ggcgaaggcg actctctggt ctgtaactga cgctgaggag cgaaagcgtg     720

gggagcgaac aggattagat accctggtag tccacgccgt aaacgatgag tgctaagtgt     780

tagggggttt ccgcccctta gtgctgcagc taacgcatta agcactccgc ctggggagta     840

cggtcgcaag actgaaactc aaaggaattg acgggggccc gcacaagcgg tggagcatgt     900

ggtttaattc gaagcaacgc gaagaacctt accaggtctt gacatcctct gacaacccta     960

gagatagggc tttcccttcg gggacagagt gacaggtggt gcatggttgt cgtcagctcg    1020

tgtcgtgaga tgttgggtta agtcccgcaa cgagcgcaac ccttgatctt agttgccagc    1080

attcagttgg gcactctaag gtgactgccg gtgacaaacc ggaggaaggt ggggatgacg    1140

tcaaatcatc atgcccctta tgacctgggc tacacacgtg ctacaatgga cagaacaaag    1200

ggctgcgaga ccgcaaggtt tagccaatcc cacaaatctg ttctcagttc ggatcgcagt 1260

ctgcaactcg actgcgtgaa gctggaatcg ctagtaatcg cggatcagca tgccgcggtg 1320

aatacgttcc cgggccttgt acacaccgcc cgtcacacca cgagagtttg caacacccga 1380

agtcggtgag gtaaccttta tggagccagc cgccgaa 1417

<210> 2
<211> 1018
<212> DNA
<213> Artificial Sequence

<220>
<223> gyrB gene sequence amplicon

<220>
<221> misc_feature
<222> (860)..(860)
<223> n is a, c, g, or t

<400> 2
ctcgttgtta gataaaattt tatctagtcg ggctttttca acgtttagga tcttccctct 60

taacggtaag atcgcttgga aatgtcgatc acgaccttgc ttagcagatc cgcccgcaga 120

atctccctct acgatataaa gctcagagat ggaaggatct ttagaagaac agtctgccag 180

tttcccaggc aagctagaga cttccagtgc acttttacgt cttgtcagct cacgtgcctt 240

tttggcagcc attcttgcac gagctgccat cacacctttc tccacaattt tctttgcagc 300

atcagggttc tctaaaagga atttctcaag tgcttcggag aagagggagt cggtaatggt 360

tcttgcttct gagttaccga gctttgtctt cgtttgtcct tcgaattgag ggtctggatg 420

tttgatagag ataatggctg ttaagccttc tcgtacatct tcaccgctca aattcgagtc 480

accatctttg aatacgccat ttttacgagc ataatcattg atcacacgcg tcagaccggt 540

tttaaagcct gcttcgtgtg ttccgccttc atatgtgttg atgttgttgg cgaaagaata 600

aatattgctt gtataggaat cgttgtattg cagtgcaact tcaacggtga ttccgtcttt 660

ttcaccctcg atgtaaactg gttcttcatg aacgacttcc ttcgagcgat tcaaatgttc 720

tacatagctc ttaataccgc cttcatagca gtattcgttt cttcgctctt tgccttcacg 780

taagtcttca atgatgatgt tgacgccttt tgttaagaaa gctaactcac gtacacggtt 840

agcaagtgtg tcgtaatcan attcaatggt ttcagtgaaa atttctggat ctggcacaaa 900

atgagtggtt gtccctgtta catctgtttc accaatgatc tctaaatctc caactggaac 960

accgcgtttg aattgttgat aatgaatttt cccatcacgg tatacggtca cgtctaaa 1018

## Description

### Field of the Invention

The invention relates to a seaweed-derived zootechnical feed additive. More specifically, the feed additive comprises spores of *Bacillus altitudinis,* which can be fed to monogastric animals and mammals to improve growth in their offspring.

### Background to the Invention

In commercial pig production, feed contributes up to 70% of total production costs. Such costs can be compounded by poor feed conversion caused by sub-optimal nutrition, infection, stress and sub-optimal weight gain. This often necessitates costly dietary supplementation on a per animal basis, which is expensive and sometimes not efficacious. Furthermore, the EU prohibition of routine in-feed antibiotic use (in 2006) and supplementation with pharmacological levels of zinc oxide (by 2022) necessitates the development of alternative sustainable treatments and strategies to support development of a healthy piglet intestinal microbiota and optimal gut health. The availability of a clean-label sow feed additive that promotes robust and durable piglet growth would mean a significant increase in the output and value of saleable meat for commercial pig producers. The need for feed additives which can act as a replacement for pharmacological levels of zinc oxide is evidenced by the Zero Zinc Summit held in Denmark in June 2019, at which there were over 450 attendees.

Any of the EU-approved probiotics for sows, *i.e.* Bonvital (Lactosan GmbH & Co; *Enterococcus faecium*), Bioplus 2B (Christian Hansen; *Bacillus subtilis* and *Bacillus licheniformis*), Biosprint (Prosol; *Saccharomyces cerevisiae*), Calsporin (Asahi Calpis Wellness Co., Ltd; *Bacillus subtilis*), Clostat (Kemin; *Bacillus subtilis*) and Levucell SC (Lallemand Animal Nutrition; *Saccharomyces cerevisiae*), only demonstrate effects in the offspring during the early post-weaning period. These products were approved for use with sows for the full reproductive cycle, *i.e.* for the entire gestation period + ∼28 days of lactation, and this lengthy administration period adds cost for the farmer. Three of the six products listed above are not spore formers and so would not be as resistant to drying and feed pelleting temperatures or as stable long-term in dried form.

Feeding piglets with probiotics to achieve weight gain has been attempted before, with little success (Caisin and Harea, Animal Science and Biotechnologies, vol. 43(1), pp. 20-25 (2010)).

It is an objective of the present invention to overcome at least one of the above-mentioned problems.

### Summary of the Invention

The invention relates to a novel *Bacillus altitudinis* probiotic feed additive with anti-*E. coli* activity that addresses the challenge of maintaining the growth performance and herd health targets required for cost-effective pig production, without the use of in-feed antibiotics or pharmacological levels of zinc oxide. The proposed benefits include improved growth performance and increased resistance to disease especially those caused by *E. coli, e.g.* post-weaning diarrhoea.

There is provided an isolated modified strain of a *Bacillus altitudinis* strain having a gyr B amplicon (fragment) sequence and/or a 16S rRNA amplicon (fragment) defined by SEQ ID NO: 1 and 2, respectively.

In one aspect, there is provided a cell extract or supernatant of an isolated modified strain of a *Bacillus altitudinis* strain having a gyr B amplicon (fragment) sequence and/or a 16S rRNA amplicon (fragment) defined by SEQ ID NO: 1 and 2, respectively.

In one aspect, there is provided a composition comprising the isolated modified *Bacillus altitudinis* strain having a gyr B amplicon (fragment) sequence and/or a 16S rRNA amplicon (fragment) defined by SEQ ID NO: 1 and 2, respectively, or the cell extract or supernatant of the isolated modified *Bacillus altitudinis* strain having a gyr B amplicon (fragment) sequence and/or a 16S rRNA amplicon (fragment) defined by SEQ ID NO: 1 and 2, respectively.

According to an aspect of the present invention, there is provided, as set out in the appended claims, an isolated *Bacillus altitudinis* strain as deposited with the National Collection of Industrial and Marine Bacteria under the Accession No. NCIMB 43558 on 27/01/2020 (hereafter "strain of the invention" or "deposited strain). This parent strain can be made antibiotic resistant by simple manipulation of its genome using techniques well-known to the skilled person. For example, the strain of the invention can be modified to be rifampicin resistant.

The invention also relates to a cell extract, a supernatant, spores or cell material derived from the isolated strain.

The invention also provides a composition comprising the isolated strain, or a cell extract, a supernatant, spores or cell material derived from the isolated strain.

The composition may be a pharmaceutical composition and may include a suitable pharmaceutical excipient. The composition may be provided in a unit dose form suitable for oral and/or topical administration, *i.e.* a tablet, a capsule, a pellet, freeze-dried granules or powder, spray-dried granules or powder, nanoparticles, microparticles or in a liquid form.

The composition may be a food or beverage product, or a nutritional supplement.

The composition may comprise a prebiotic material, such as inulin, galacto-oligosaccharides, fructo-oligosaccharides, lactulose, mannose, maltose, mannan-oligosaccharides; malto-oligosaccharides, isomaltulose, palatinose, xylan, xylo-oligosaccharides, arabinoxylo-oligosaccharides, soy, soy polysaccharides, chito-oligosaccharides, cello-oligosaccharides, raffinose oligosaccharides, galactosyllactose, yeast cell wall extracts, seaweed extracts, laminarin, fucoidan, pectin, cellulose, resistant starch, plant polyphenols, polydextrose, β-glucans and the like.

The composition may comprise an additional probiotic bacterium such as a *Bacillus* species, a *Lactobacillus* species, a *Clostridium* species, an *Enterococcus species, E. coli,* a *Pediococcus* species, and the like, and/or probiotic yeast, such as *Saccharomyces cerevisiae* or S. *boulardii,* and the like.

The strain in the composition may be viable or non-viable and may comprise a strain extract *(i.e.* bacterial cell lysate) or supernatant derived from the strain. The extract or supernatant may be in any physical form, for example liquid or dried.

The composition may comprise at least 10⁶ cfu per g of composition. In one aspect, the composition may comprise 1 x 10⁹ spores/ml or cfu/ml. Typically, the composition may comprise between 1 x 10⁶ to 1 x 10¹⁰ spores/ml or cfu/ml. For example, 1 x 10¹⁰ spores/ml or cfu/ml, 1 x 10⁷ spores/ml or cfu/ml, 1 x 10⁸ spores/ml or cfu/ml, 1 x 10⁹ spores/ml or cfu/ml, or 1 x 10¹⁰ spores/ml or cfu/ml.

The composition may be solid or liquid. The composition may comprise a carrier for oral delivery. The carrier may be in the form of tablet, capsule, powder, granules, microparticles or nanoparticles. The carrier may be configured for targeted release in the intestine (*i.e.* configured for gastric transit and ileal release).

The composition may be dried or lyophilised.

In one aspect, when the composition is suitable for topical or environmental administration, the composition comprises a carrier for topical or environmental administration selected from the group comprising powder, granules, microparticles, nanoparticles, a cream, a spray, spores or liquid.

There is also provided a (pharmaceutical) composition for use in a method of increasing weight gain in offspring of a monogastric animal fed or exposed to the composition.

In one aspect, the (pharmaceutical) composition described above can be used in a method of improving the health of an individual.

There is also provided a method of increasing weight gain in an offspring of a lactating mammal, the method comprising providing the lactating mammal with the (pharmaceutical) composition described above during late gestation and during the lactating period.

In one aspect, the mammal is fed the composition for about the final 14 days of gestation and for about the first 28 days of the lactating period.

In one aspect, the composition is applied to the body of the lactating mammal for about the final 14 days of gestation and for about the first 28 days of the lactating period.

In one aspect, the composition is dispersed into the environment of the lactating mammal for about the final 14 days of gestation and for about the first 28 days of the lactating period.

In one aspect, the lactating mammal is selected from a human, primates, non-human primates, farm or farmed animals (such as pigs, horses, goats, sheep, cows (including bulls, bullocks, heifers etc.), donkey, reindeer, etc.), veterinary mammals and animals (such as dogs, cats, rabbits, hamsters, guinea pigs, mice, rats, ferrets, etc.), and mammals and animals kept in captivity (such as lions, tigers, elephants, zebras, giraffes, pandas, rhino, hippopotamus, etc.).

There is also provided a method of increasing weight gain in an offspring of a monogastric animal, the method comprising feeding the monogastric animal with the (pharmaceutical) composition described above during late gestation and for about the first 28 days following birth.

In one aspect, the composition is applied to the body of the monogastric animal for about the final 14 days of gestation and for about the first 28 days following birth.

In one aspect, the composition is dispersed into the environment of the monogastric animal for about the final 14 days of gestation and for about the first 28 days following birth.

In one aspect, the monogastric animal is selected from a human, primates, non-human primates, non-ruminant farm or farmed animals (such as pigs, horses, donkey, chicken, turkey, ducks, fish (salmon, trout, abalone), etc.), non-ruminant veterinary mammals and animals (such as dogs, cats, rabbits, hamsters, guinea pigs, mice, rats, ferrets, etc.), and non-ruminant mammals and animals kept in captivity (such as lions, tigers, elephants, zebras, pandas, rhino, hippopotamus, etc.).

There is also provided a method of increasing weight gain in an offspring of a bird, the method comprising injecting the (pharmaceutical) composition described *in ovo* prior to hatching.

The bird is typically selected from an egg-laying hen, a wild turkey, a farmed turkey, a Guinea fowl, a goose, a duck, a canary, a budgerigar, a parrot, and the like.

The invention also provides a method of producing a supernatant from an isolated *Bacillus altitudinis* strain comprising a step of culturing the isolated strain and separating the supernatant from the strain.

The invention also provides a method of producing an extract from an isolated *Bacillus altitudinis* strain comprising a step of lysing the cell and separating the cell extract from lysed cell material.

The invention also provides a supernatant or bacterial material or extract (for example a cell lysate) formed according to the method of the invention.

Thus, a composition comprising spores from the *B. altitudinis* strain of the claimed invention can be used to improve the nutritional quality of colostrum (increased protein content), positively modulate the histology parameters of the intestine and modulate the intestinal and/or colostrum microbiome.

*"Bacillus altitudinis* strain" refers to the strain of bacteria deposited with the National Collection of Industrial and Marine Bacteria (Ferguson Building, Craibstone Estate, Bucksburn, Aberdeen AB219YA, UK) under the Accession No. NCIMB 43558 on 27/01/2020. The Deposit was made by the Waterford Institute of Technology, and having an address of Waterford Institute of Technology, Waterford, Ireland. The term is intended to include the strain in a viable or non-viable form, or mixtures of viable and non-viable bacteria. The strain may be provided in any format, for example as a liquid culture (or supernatant derived from a liquid culture), or cell material, spores or extract derived from the strain or a culture of the strain, or in a dried or freeze-dried format. The invention may also employ growth media in which the strain of the invention was grown, or cell lysates generated using the strain of the invention. The term also includes mutants, modified strains and variants of the deposited strain that are substantially identical, genetically and phenotypically, to the deposited strain and retain the activity of the deposited strain, such as introducing an antibiotic resistant gene (for example, rifampicin) to the strain. Thus, the term includes derivatives of the strain that have been genetically engineered to modify the genome of the strain, typically without fundamentally altering the functionality of the organism, for example engineered for heterologous expression of a nucleic acid, or engineered to overexpress an endogenous gene, or engineered to silence a gene, to produce a recombinant or transformed strain of the invention. Genetic modifications may be carried out using recombinant DNA techniques and reagents, including gene editing technologies such as CRISP-Cas9 techniques (see below). The term also includes variants of the strain having natural or spontaneous genetic alterations. The term is also intended to encompass variant strains obtained by serial passage of the isolated strain of the invention. The variant generally has a gyr B amplicon (fragment) sequence and/or a 16S rRNA amplicon (fragment) that is identical or substantially identical with the deposited strain, for example at least 99.1%, 99.2%, 99.3%, 99.4%, 99.5%, 99.6%, 99.7%, 99.8% or 99.9% identical to the deposited strain. Sequence homology can be determined using an online homology algorithm "BLAST", publicly available at http://www.ncbi.nlm.nih.gov/BLAST/. The sequence of the gyr B amplicon (SEQ ID NO: 1) and the sequence of the 16S rRNA amplicon (SEQ ID NO: 2) for the Deposited Strain is provided in Annex 1 below.

### Dosage

It is preferable that the strain or composition is administered at least once per day over a treatment period of at least 6 weeks, and preferably for at least 7, 8, 9, 10, 11, 12, 13, 14, 15, 16, 17, 18- or 20-week periods. Compositions of the invention generally comprise between 10³ and 10¹² cfu or spores of the strain of the invention per g or per ml of the composition, respectively. In one embodiment, the composition comprises 10³ and 10¹² cfu or spores, or 10⁴ and 10¹² cfu or spores, or 10⁶ and 10¹⁰ cfu or spores of the strain of the invention per g or per ml of the composition, respectively. A daily dose generally comprises between 10³ and 10¹² cfu or spores per g or per ml of the strain, respectively. In one embodiment, the daily dose comprises 10³ and 10¹² cfu or spores, or 10⁴ and 10¹² cfu, or 10⁶ and 10¹⁰ cfu or spores per g or per ml of the strain, respectively. In one aspect, the daily dose can be administered orally or by applying the composition to the environment in which the individual is residing, or to the skin or feathers of the adult animal or the offspring. For example, the composition could be applied to the udder of the lactating mammal or onto the offspring themselves once they are born. Another example is where the composition is applied to the environment within which the individual is residing.

After administering the strain described herein to gestating/lactating sows for a 6-week period (*i.e.* during the last 2 weeks of gestation and the ∼28 days of lactation), residual growth benefits in offspring were observed during the finisher period and at slaughter, at which point improved carcass weight was found, which has financial benefits for pig producers.

The strain can also be used in other monogastric animal species, for example, chickens, as, like pigs, they are farmed intensively with the use of in-feed antibiotics.

### Definitions

In the specification, the term "healthy" should be understood to mean where the monogastric animal or mammal has no underlying medical condition, infection, inflammatory response, condition or otherwise occurring.

In the specification, the term "infection" or "infectious condition" should be understood to mean infections or diseases caused by microorganisms or microbes such as viruses, bacteria, fungi, protozoa and helminths. Examples of infections include septicaemia, meningitis, eye infections, tuberculosis, upper respiratory tract infections, pneumonia, gastroenteritis, nail and skin infections and the like. Common bacteria that cause disease and infections include *Staphylococcus, Streptococcus, Pseudomonas, Clostridium difficile, Salmonella, Campylobacter, Escherichia coli* and *Listeria monocytogenes.*

In the specification, the term "inflammatory condition" should be understood to mean immune-related conditions resulting in allergic reactions, myopathies and abnormal inflammation and non-immune related conditions having causal origins in inflammatory processes. Examples include acne (in humans), asthma, autoimmune conditions, autoinflammatory condition, celiac disease (in humans), chronic prostatitis, colitis, diverticulitis, glomerulonephritis, inflammatory bowel diseases, lupus, rheumatoid arthritis, vasculitis, cancer, heart disease, and the like.

In the specification, the term "individual" should be understood to mean all monogastric animals and mammals and pre-ruminants (an animal that does not yet chew the cud), for example, a human, primates, non-human primates, farm or farmed animals (such as pigs, horses, goats, sheep, cows (including bulls, bullocks, heifers *etc.*), donkey, reindeer, chicken, turkey, ducks, fish (salmon, trout, abalone, *etc*.), veterinary mammals and animals (such as dogs, cats, rabbits, hamsters, guinea pigs, mice, rats, ferrets, *etc*.), and mammals and animals kept in captivity (such as lions, tigers, elephants, zebras, giraffes, pandas, rhino, hippopotamus, *etc*.), and other animals, mammals and higher mammals for which the use of the invention is practicable.

In the specification, the term "non-ruminant animal" should be understood to mean any mammal excluding cattle, all domesticated and wild bovines, goats, sheep, giraffes, deer, gazelles, and antelopes.

In this specification, the term "biological sample" should be understood to mean blood or blood derivatives (serum, plasma etc), urine, saliva, faecal samples, gut digesta, gut tissue, colostrum, milk or cerebrospinal fluid.

In the specification, the term "treatment" should be understood to mean prohibiting, preventing, restraining, and slowing, stopping or reversing progression or severity of a disease or condition associated with inflammatory or non-inflammatory diseases, condition or infections.

In the specification, the term "improving growth" should be understood to mean improving the weight gain in offspring of individuals where the individuals have been exposed to the strain of the present invention. By using the term "exposing", we mean that the adult individuals have been fed the strain of the present invention, or have had their body parts (such as udders, teats, feathers, brooding patch, skin, fur, *etc*.) sprayed with a composition comprising the strain, or the environment where the adults and offspring are residing is sprayed with the composition. While not bound by the theory, typically, there is transference of the strain from the adult to the offspring or from the environment to the offspring.

### Brief Description of the Drawings

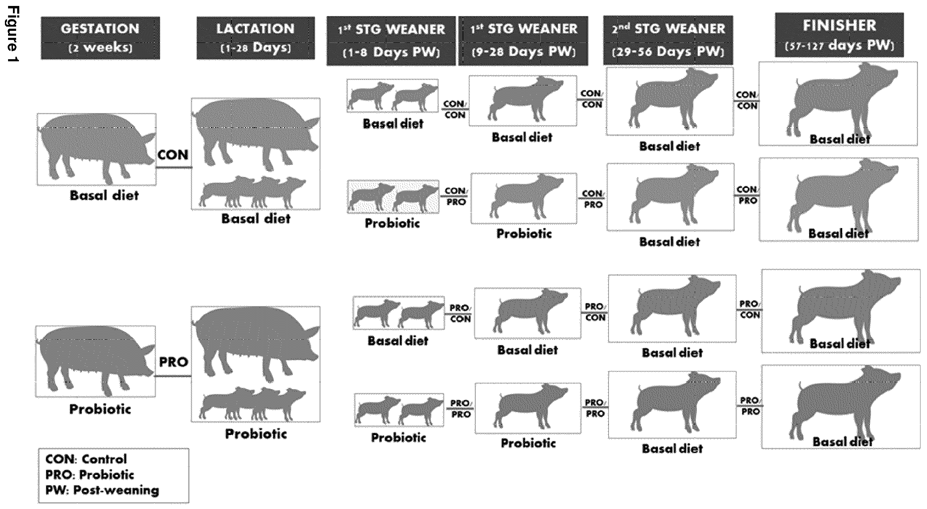

The invention will be more clearly understood from the following description of an embodiment thereof, given by way of example only, with reference to the accompanying drawings, in which:-
**Figure 1** illustrates the trial layout for use of the deposited strain of the invention in pigs.
**Figure 2** illustrates the duodenal morphology of (A) a piglet euthanized on D7-D8 post-weaning, born to a sow fed a basal diet supplemented with spores of the deposited strain of the claimed invention from D100 of gestation until weaning (D26 of lactation) compared to that of (B) a piglet born to a sow fed a basal (control) diet. The difference in villous height is indicated.

### Detailed Description

### Materials and Methods

Ethical approval was granted by the Teagasc Animal Ethics Committee (approval no. TAEC148/2017) and the project was authorised by the Health Products Regulatory Authority (project authorisation no. AE19132/P066). The experiment was conducted in accordance with Irish legislation (SI no. 543/2012) and the EU Directive 2010/63/EU for animal experimentation. A total of 24 healthy sows were selected on day 100 of gestation, blocked on parity, body weight and back fat and randomly assigned to 2 experimental treatment groups (12 sows/group) as follows:
- CON: control, basal gestation/lactation diet (n=12)
- PRO: basal gestation/lactation diet supplemented with spores of the B. *altitudinis* strain of the claimed invention [∼4 x 10⁹ spores/sow/day during gestation and ∼1.2 X 10¹⁰ spores/sow/day during lactation (n=12)]

A total of 144 weaned piglets (∼28 d of age) from the above 24 sows (n=12 sows/trt *i.e.* 6 piglets per sow) were blocked on litter origin, sex and body weight, and randomly assigned to 4 experimental treatment groups following a 2 x 2 factorial arrangement where the main factors were probiotic inclusion in the sow diet (yes vs. no) and in 1^{st} stage weaner diets (yes vs. no). The four treatment groups were as follows:
- CON/CON: piglets from CON sows fed basal weanling diet (n=18 pens)
- CON/PRO: piglets from CON sows fed basal weanling diet supplemented with spores of the *B. altitudinis* strain of the claimed invention (∼1 X 10⁹ spores/piglet/day) (n=18 pens)
- PRO/CON: piglets from PRO sows fed basal weanling diet (n=18 pens)
- PRO/PRO: piglets from PRO sows fed basal weanling diet supplemented with spores of the *B*. *altitudinis* strain of the claimed invention (∼1 X 10⁹ spores/piglet/day) (n=18 pens)

The probiotic comprising the *B. altitudinis* strain of the present invention was fed at the optimum dose, as determined in a previous trial and also taking into account doses used for commercial probiotics (Table 1). During the gestation period, sows were given ∼4 x 10⁹ spores/day. For lactating sows, ∼1.2 X 10¹⁰ spores/day were provided (Table 1). For 1^{st} stage weaning piglets (up to day 28 post weaning (pw)), ∼1 X 10⁹ spores/day were given (Table 1). All pigs were followed to the end of the finisher period.

Feed and water were provided on an *ad libitum* basis during lactation and at between 2-3 kg/day for the 10 days prior to parturition. Sows were fed with a standard gestation diet from day 100 of gestation to farrowing, followed by a standard lactation diet for 28 days (until weaning). The composition of the experimental diets is shown in Tables 2 and 3. In the case of PRO sows, the diet was supplemented with spores of the B. *altitudinis* strain of the claimed invention from day 100 of gestation until weaning of piglets. Probiotic spores were administered daily. Spore suspensions were top dressed onto the feed as outlined below. The CON group received the same volume of sterile water top dressed onto the feed daily.

### Piglet Offspring

Piglets were penned as pairs for the first 7-8 days pw. On day 7 (20 piglets) and day 8 (20 piglets) pw, a total of 40 piglets (10 piglets per treatment) were sacrificed to determine intestinal probiotic counts and microbiota profile and to assess gut health. The paired piglets from the other pens were removed from the trial and the remaining piglets were individually housed until the end of the 2nd stage weaner period (day 55 pw). At day 56 pw, pigs were moved to finisher rooms where they were individually penned in fully slatted pens (1.81 m x 1.18m) until the end of the trial (day 127 pw).

Feed and water were provided *ad libitum.* Starter/link diet were fed for the first 28 days after weaning. Weaner diet was provided from day 29 pw, followed by finisher diet from day 57 pw until slaughter. All feed was non-medicated. The composition of the experimental diets is shown in Tables 2 and 3. During 1^{st} stage weaning, pigs were fed with enough feed in the evening to ensure that they had sufficient feed overnight but that feeders were empty in the morning so that they immediately consumed the probiotic feed. This was practiced only during the 1st stage weaning period.

Probiotic spores were administered daily (after any sampling that took place) to treatment groups (CON/PRO and PRO/PRO) during the 1^{st} stage weaner period only. A small amount of feed was put into the feeder and spore suspensions were top dressed onto the feed as outlined below. Any treatment groups not receiving probiotic (PRO/CON and CON/CON) received the same volume of sterile water top dressed onto the feed.

Growth performance of piglets was assessed by weighing pigs individually and monitoring individual feed intake in order to calculate average daily gain (ADG), average daily feed intake (ADFI), and feed conversion efficiency (FCE). Feed weigh-backs were recorded weekly and pigs were individually weighed as outlined below. Pigs were fasted for 12 hours prior to pre-slaughter weighing. Blood samples were taken at the same time points for one or more of serum biochemistry and haematological analyses to assess health.

### Hygiene Practices

### Farrowing room

All farrowing rooms were thoroughly washed and disinfected a week before moving in the sows. Then one swab (blue sponge swab soaked in neutralising buffer; TS/15-A-95, Technical Service Consultants Ltd) was taken from each room for bacterial culture to check the absence of the probiotic strain in the farrowing rooms prior to entry of the sows.

During the experiment, every precaution was taken to prevent cross-contamination between treatments. Gloves were changed between pigs, and fresh disposable overalls worn by all personnel prior to commencing sampling. Particular care was taken to prevent the probiotic spores contaminating the control sows. Probiotic-fed sows were housed separately in two rooms. A foot bath containing 1% Virkon was placed outside each room to avoid contamination between rooms. Virkon solution was freshly prepared every Monday morning and Wednesday evening, or before, should it have lost its pink colour. Gloves and overalls were removed before leaving the probiotic farrowing room and put into a waste bag for immediate disposal.

To avoid contamination with probiotic spores, collection of samples, weighing of pigs and administration of distilled water/spores was done first with control sows followed by sows supplemented with spores. All equipment e.g. weighing scales, snare used for blood sampling, *etc.,* was disinfected thoroughly with Virkon after each treatment and sampling/weighing day to prevent cross contamination at subsequent sampling.

During blood sampling, puncture wounds were cleaned with alcohol prior to and after blood sampling.

### Sample collection and parameters measured

### Sows

### Blood and Faecal samples (N=24), Weighing (N=24) and Back fat scanning (N=24)

Blood and faecal samples were collected at the start of the trial (day 100 gestation), close to farrowing (day 114 and 115 of gestation), and at weaning (D26 of lactation). Body weight and back fat of all sows was recorded at the start of the trial (day 100 gestation), at farrowing and at weaning (D26 of lactation).

### Colostrum and milk (N=24)

Colostrum (15-20 ml or as much as possible) was collected from each sow within 12 hrs of farrowing. At D14 of lactation, 40 ml of milk was collected from each sow.

### Piglets

### Blood samples

At weaning, D7/D8 pw during sacrifice, D28 pw and D57 pw, blood samples were taken from 40 piglets (10 piglets/treatment).

### Faecal samples

On day 14 of lactation, rectal swabs were taken from a subset of suckling piglets (2 piglets/sow, n=48). Thereafter, faecal samples from 40 piglets (10 piglets/treatment) were collected from the rectum at weaning, at D7 and D8 pw during sacrifice, at D27 pw and at D56 pw and naturally voided samples were collected from as many pigs as possible during the grow-finisher stage (D117 and 118 pw).

### Microbiome analysis of pig faecal, rectal and diaesta samples by 16S rRNA gene sequencing

DNA was extracted from samples using the QIAamp DNA stool minikit (Qiagen, Crawley, United Kingdom) according to the manufacturer's instructions, apart from adding a bead beating step after sample addition to the InhibitEX buffer and increasing the lysis temperature to 95°C to increase the DNA yield. Microbial profiling at phylum, family, genus and ASV levels was performed using high throughput sequencing of the V3-V4 region of the 16S rRNA gene on an Illumina MiSeq platform according to standard Illumina protocols (McCormack et al., 2017, Exploring a possible link between the intestinal microbiota and feed efficiency in pigs. Applied & Environmental Microbiology 83(15): e00380-17).

### Microbiome analysis of colostrum samples

DNA was extracted from colostrum samples using the DNeasy® PowerFood® Microbial Kit (Qiagen, Crawley, United Kingdom) according to the manufacturer's instructions. Microbial profiling was performed as outlined above for faecal/rectal/digesta samples.

### Weighing

Body weight of all piglets was recorded at birth (D0), during the lactation period (D14) and at weaning (D26) (N=144). At D7 and D8 pw, body weight was recorded prior to sacrifice (N=40). Body weight was also recorded at D14, D28, D56, D105 and D127 pw (N=72).

### Sacrifice (N=40)

On D7 and D8 pw one pig per pen from each of the 10 pens selected per treatment (n = 40) was sacrificed to collect blood, digesta and gut tissue samples.

### Carcass data (N=72)

On D127 pw, finisher pigs were slaughtered at a commercial abattoir. Carcass weight, kill out %, lean meat %, muscle depth and fat depth data were collected from the factory.

### Diet Supplementation

**Table 1: Spore doses for sows and piglets**

| **Stage** | **Concentration (probiotic spores/ml)** | **Volume** | **Total Dose (probiotic spores/day)** | **Average of Commercial Doses (probiotic spores/day)** |
|---|---|---|---|---|
| 1^{st} Stage Weaners | 1 x 10⁹ | 1ml | 1 x 10⁹ | 5.2 x 10⁸ |
| Gestating Sows | 1 x 10⁹ | 4ml | 4 x 10⁹ | 2.4 x 10⁹ |
| Lactating Sows | 1 x 10⁹ | 12mls | 1.2 x 10¹⁰ | 6.6 x 10⁹ |

**Table 2. Calculated analysis of experimental diets (g/kg)**

| **Diet Type** | **Dry Sow** | **Lact Sow** | **Starter/Link** | **Weaner** | **Finisher** |
|---|---|---|---|---|---|
| DM | 873.27 | 881.94 | 888.81 | 874.92 | 875.14 |
| Protein | 140.00 | 175.00 | 208.84 | 185.00 | 170.00 |
| Ash | 47.34 | 50.66 | 57.60 | 47.20 | 43.33 |
| Fat | 31.44 | 79.85 | 70.58 | 64.37 | 25.66 |
| Fibre | 80.00 | 29.46 | 25.38 | 34.72 | 36.88 |
| Starch + Sugar | 425.76 | 434.57 | 424.67 | 435.52 | 480.76 |
| NDF | 213.41 | 121.37 | 87.32 | 125.46 | 132.31 |
| ADF | 108.59 | 37.56 | 31.49 | 46.20 | 42.49 |
| Moisture | 126.73 | 118.06 | 111.19 | 125.08 | 124.86 |
| DE | 13.20 | 15.20 | 15.00 | 14.50 | 13.81 |
| NE IFIP | 8.83 | 10.90 | 9.77 | 10.55 | 9.80 |
| Lysine | 8.22 | 11.50 | 15.00 | 13.00 | 11.00 |
| Methionine | 2.70 | 3.83 | 5.76 | 4.59 | 3.52 |
| Cystine | 2.68 | 3.07 | 3.29 | 3.30 | 3.15 |
| Methionine +Cysteine | 5.38 | 6.95 | 9.08 | 7.89 | 6.68 |
| Threonine | 5.46 | 8.32 | 10.06 | 8.60 | 7.69 |
| Tryptophan | 1.69 | 2.78 | 3.35 | 2.60 | 2.30 |

**Table 3. Vitamin trace mineral inclusion levels (per tonne finished diet)**

| | **Dry Sow** | **Lactating Sow** | **Starter/link** | **Weaner** | **Finisher** |
|---|---|---|---|---|---|
| Copper sulphate. 7H₂0, g | 60 | 60 | 620 | 620 | 60 |
| Ferrous sulphate monohydrate, g | 200 | 200 | 450 | 450 | 200 |
| Manganese oxide, g | 80 | 80 | 60 | 60 | 80 |
| Zinc oxide, g | 100 | 100 | 150 | 150 | 100 |
| Potassium iodate, g | 1 | 1 | 1 | 1 | 1 |
| Sodium selenite, g | 0.4 | 0.4 | 0.6 | 0.6 | 0.4 |
| Vitamin A, miu | 10 | 10 | 6 | 6 | 10 |
| Vitamin D₃, miu | 1 | 1 | 1 | 1 | 1 |
| Vitamin E, * 1,000 iu | 100 | 100 | 100 | 100 | 100 |
| Vitamin K, g | 2 | 2 | 4 | 4 | 2 |
| Vitamin B₁₂, mg | 15 | 15 | 15 | 15 | 15 |
| Riboflavin, g | 5 | 5 | 2 | 2 | 5 |
| Nicotinic acid, g | 12 | 12 | 12 | 12 | 12 |
| Pantothenic acid, g | 10 | 10 | 10 | 10 | 10 |
| Choline chloride, g | 500 | 500 | 250 | 250 | 500 |
| Biotin, mg | 200 | 200 | - | - | 200 |
| Folic acid, g | 5 | 5 | - | - | 5 |
| Vitamin B₁, g | 2 | 2 | 2 | 2 | 2 |
| Vitamin B₆, g | 3 | 3 | 3 | 3 | 3 |
| Endox, g | - | - | 60 | 60 | - |
| | | | | | |
| | | | | | |

| **Inclusion levels of minerals** | | | | | |
|---|---|---|---|---|---|
| Copper | 15 | 15 | 155 | 155 | 15 |
| Iron | 70 | 70 | 90 | 90 | 70 |
| Manganese | 62 | 62 | 47 | 47 | 62 |
| Zinc | 80 | 80 | 120 | 120 | 80 |
| Iodine | 0.6 | 0.6 | 0.6 | 0.6 | 0.6 |
| Selenium | 0.2 | 0.2 | 0.3 | 0.3 | 0.2 |

### Compositional analysis of sow colostrum and milk samples

Colostrum and milk samples were defrosted at room temperature. When fully thawed, samples were mixed by inverting several times to disrupt settled solids and mixed well. The volume of each sample was recorded prior to decanting into 50 ml tubes on ice. Sterile water was added to bring up the volume to 40 ml. Tubes were mixed thoroughly and kept on ice. Each sample was analysed in duplicate for total solids, lactose, fat, protein, true protein and casein B content by near-infrared absorption using a Bentley Dairyspec FT (Bentley Instruments, Inc., Chaska, MN, USA). Data were recorded as % (g/100g), taking the dilution factor into account.

### Intestinal histology

Duodenal, jejunal, and ileal tissue samples were rinsed in phosphate buffered saline immediately post-harvest and placed in No-Tox, an alcohol/aldehyde fixative (Scientific Device Lab, Des Plaines, IL) on a shaker for 48 hr. Samples were then dehydrated through a graded alcohol series, cleared with xylene and embedded in paraffin wax. Tissue samples were sliced into 5 micrometre sections using a microtome (Leica RM2135, Wetzlar, Germany), mounted on microscope slides and stained with hematoxylin and eosin for determination of gross morphological parameters of intestinal structure (villus height and width and crypt depth and width). For each pig, 10 villi and 10 crypts were measured on five fields of view, where villi were attached to the lumen, and the means were utilised for statistical analysis. The goblet cell number was determined by periodic acid-Schiff staining. Positively stained periodic acid-Schiff cells were enumerated on 10 villi/sample, and the means were utilised for statistical analysis.

### Statistical analysis:

A power calculation was performed to determine the minimum number of observations required to detect effect sizes, using a statistical power of 80%, an α level at 5% and standard deviation of similar variables of interest from previously published studies. The power calculation indicated that 12 sows per treatment were required to see a difference of 2.5mm in back-fat and 18 piglets were required to see a 1.5 log₁₀ CFU difference in selected microbial counts between treatments.

The experiment was a 2 x 2 factorial arrangement, including the main effects maternal treatment (Control or Probiotic spore supplementation) and post-weaning treatment (Control or Probiotic spore supplementation). All data were analysed using the MIXED procedure, unless otherwise stated, in SAS® 9.4 (SAS Institute, Inc., Cary, NC, US). The model included maternal treatment and post-weaning treatment and their interaction. Where required, data were analysed as a repeated measure with sampling day as the repeated variable. Simple main effects were obtained using the 'slice' option in SAS.

The sow/litter was the experimental unit for sow performance and the individual pig was the experimental unit for analysis of pre- and post-weaning pig growth performance, carcass characteristics, haematology, small intestinal morphology and microbiota data. The normality of scaled residuals was investigated using the Shapiro-Wilk and Kolmogonov-Smirnov tests within the UNIVARIATE procedure of SAS. Differences in least square means were investigated using the t-test after Tukey adjustment for multiple comparisons. Degrees of freedom were estimated using Satterthwaite adjustment.

For sow performance, litter size and pre-weaning mortality data, block was used in the model and sow was used as a random effect. Pre-weaning and post-weaning performance were analysed as repeated measures, with sex used in the model while birth weight or weaning weight was included as a covariate for the analysis of pre-weaning and post-weaning data, respectively. For carcass quality, body weight at weaning and sex were used as covariates. Counts of the strain of the invention were analysed as repeated measurements, with the individual animal as a random variable and the measurement taken on D0 used as a covariate. Haematological parameters were analysed as repeated measurements, with gender and the measurement taken on D0 used as covariates. The haematological parameters that were not normally distributed were further analysed to find the best fitting distribution using the GLIMMIX procedure in SAS, using a gamma distribution. For these variables, the ilink function was used to back-transform the data to the original scale. The small intestinal morphological data were analysed using bodyweight at weaning and gender as covariates.

The results are presented as the least square means together with the pooled standard error. Differences were considered significant at P < 0.05 and as tendencies at P > 0.05 but less than P < 0.10.

### Evaluate the effects of spores of the probiotic strain B. altitudinis of the claimed invention administered to piglets during 1st and/or 2nd stage weaning

The objective of this study was to evaluate the effects of spores of the deposited strain *B. altitudinis* of the claimed invention administered during 1^{st} and/or 2nd stage weaning on growth performance, health indicators and gut microbiota composition of pigs in comparison to an antibiotic (AB) zinc oxide (ZnO) combination. A total of 80 piglets (40 male and 40 female) were selected one day prior to weaning, blocked by sex, weight and litter origin, and randomly assigned to one of five experimental treatments as follows: 1) Negative control (no spores of the deposited strain *B. altitudinis* of the claimed invention) fed from D0-56 pw (Neg/Neg); 2) Control diet with spores of the deposited strain *B. altitudinis* of the claimed invention fed from D29-56 pw (Neg/Pro); 3) Control diet with spores of the deposited strain *B. altitudinis* of the claimed invention fed from D0-28 pw (Pro/Neg); 4) Control diet with spores of the deposited strain B. *altitudinis* of the claimed invention fed from D0-56 pw (Pro/Pro) and 5) Control diet containing ZnO at 2500 mg Zn/kg feed and AB (200 mg Apramycin /kg feed) from D0-28 pw. The spores of the deposited strain *B. altitudinis* of the claimed invention were fed at ∼1 x 10⁹ spores/day from D0 to 28 pw and ∼2 x 10⁹ spores/day from D29 to 56 pw. All pigs were followed until the end of the finisher period (D106 pw) to monitor residual effects of treatments.

### Results and Discussion

The concept is a novel seaweed-derived zootechnical feed additive. It comprises spores, supernatant, cell material of the deposited strain of the invention, which when fed to sows during late gestation and lactation, resulted in improved growth in the offspring.

A feeding trial compared the effects of this *Bacillus* feed additive on progeny growth when administered to sows over a -6-week period (last 2 weeks of gestation and the ∼28 days of lactation) against a control treatment (feed without the additive). Sows and offspring from both groups were continuously monitored and biological samples were collected from offspring at regular intervals from birth to slaughter. It was found that piglets born to sows fed the additive demonstrated faecal shedding of the strain while suckling, thereby demonstrating transfer of the microbial additive from sow to offspring. These piglets also exhibited the following benefits:
- Numerically reduced pre-weaning mortality (11.3 vs 14.7% in offspring from control sows; P = 0.425)
- Better feed conversion ratio (FCR) for the first 14 days pw (1.28 vs 1.45 in offspring from control sows; P < 0.001). Commercially, this is important as feed contributes up to 70% of total production costs. Based on the fact that pigs eat on average ∼3.0 kg feed in the first 14 days pw, the 0.17 reduction in FCR means that pigs from probiotic-supplemented sows will eat ∼510 g less feed during this period which will save ∼46 cents/pig given the price of feed is ∼€0.895/kg. This represents a considerable cost-saving for farmers. However, and much more important, is the fact that an improvement in FCR at this time can be considered a proxy for improved intestinal health at weaning which can be correlated with increased lifetime growth.
- Numerical improvements in body weight were found up to day 56 pw in offspring from probiotic-supplemented sows (+0.2, +0.6 and +0.6 kg on day 14, 28 and 56 pw, respectively; P>0.05).
- After this, offspring were followed through the finisher stage to slaughter to monitor residual growth effects. Significant improvements in finisher pig live-weight were observed with offspring from probiotic-supplemented sows 3.5 kg heavier at 105 days pw (P = 0.013) and 3.5 kg heavier at day 127 pw (P = 0.012).
- Due to an increase in kill out yield for offspring from probiotic-fed sows (P=0.003) the carcass weight of offspring from probiotic-supplemented sows was 4.1 kg heavier than that of offspring from control sows (P = 0.03). This translates to a financial benefit of €6.31 more per pig (at a 5-year average pig price of €1.54/kg carcass weight). This equates to €169.74 more income per sow per year, based on the fact that a sow produces an average of 26.9 piglets/year (Teagasc, 2018). Based on a typical 500-sow unit, this equates to a benefit of €84,870 a year from probiotic supplementation of sows.
- Benefit to cost analyses were also performed using costs from some competitor probiotics as we do not know what the production costs will be for our probiotic at this stage. Two probiotics approved for use in sows were selected; Calsporin, which is relatively cheap at €1.50/tonne of finished feed, and Levucell, which is more expensive, at €5/tonne of finished feed. These costs and average sow feed intake data were firstly used to estimate the cost of feeding probiotic during the 6-week feeding period that was used (*i.e.* 2 weeks gestation + 4 weeks lactation) OR the entire reproductive cycle, *i.e.* entire gestation and lactation period (which is what these probiotics are recommended/approved for) as follows:
   - Based on the cost of Calsporin, it would cost 82c per sow per year to feed probiotic for the 6-week period and €2.10 for the full reproductive cycle.
   - Based on the cost for Levucell, it would cost €2.73 per sow per year for a 6-week administration period and €7 per sow for the full reproductive cycle.

Bearing in mind that feeding the probiotic of the claimed invention to sows would generate €169.74 more per sow per annum as outlined above, the benefit to cost was then estimated as follows:
- Based on the cost of Calsporin and probiotic administration for 6 weeks, benefit to cost = 207:1, whereas if fed for the whole reproductive cycle, it would be 81:1. This essentially means that if adopting the 6-week administration period, for every €1 spent, a farmer could expect to get €207 back and for the full reproductive cycle, this would be €81.
- Based on the cost of Levucell, this would be 62:1 for the 6-week period and 24:1 for the full reproductive cycle i.e. for every €1 spent, a farmer would expect to get back €62 or €24, respectively.

There was no impact of probiotic supplementation to offspring on body weight, ADG, feed intake or FCR, post-weaning or during the finisher period and there was no impact on carcass weight or quality
In piglets born to sows fed probiotic, faecal probiotic shedding was detected while the piglets were suckling (see Table 4 below), even though probiotic had not been administered to the piglets themselves, demonstrating transfer from sow to offspring, which could offer a potentially cost-effective means of probiotic delivery to piglets in early life. This group of piglets shed probiotic at day 14 and 27 of age (*i.e.* while still suckling the sow). In fact, the faecal probiotic count in these piglets at day 27 of age was log₁₀ 4.79 cfu/g while in piglets supplemented with probiotic for the entire 1^{st} stage weaner period it was log₁₀ 5.9 cfu/g at day 28 pw 9. This indicates that the probiotic had transferred successfully from the sow to offspring and as such this could prove to be a cost-effective means of probiotic delivery to piglets early in life.

**Table 4: Counts of the deposited Bacillus altitudinis strain of the claimed invention in faeces (log₁₀ cfu/g) of sows and their piglets during gestation and lactation, where sows were fed a basal diet (Control) or a basal diet supplemented with spores of the deposited strain B. altitudinis (Probiotic) from day (D) 100 of gestation until weaning (D26 of lactation)^{1,2}**

| | Treatment | | | | P-value | |
|---|---|---|---|---|---|---|
| Time point | Control | Probiotic | SEM³ | Treatment | Day | Treatment x Day |
| **Sows** | | | | | | |
| N | 12 | 12 | | | | |
| D100 of gestation | 3.00 | 3.00 | - | - | | - |
| D115 of gestation | 3.04 | 5.87 | 0.039 | **<0.001** | | |
| D13 of lactation | 3.00 | 6.34 | 0.039 | **<0.001** | | |
| Weaning (D26 of lactation) | 3.00 | 6.13 | 0.039 | **<0.001** | | |
| Mean | 3.01 | 6.11 | 0.023 | **<0.001** | **<0.001** | **<0.001** |
| | | | | | | |

| **Piglets during lactation** | | | | | | |
|---|---|---|---|---|---|---|
| N | 24 | 24 | | | | |
| D14⁴ | 3.00 | 3.47 | 0.075 | **<0.001** | | |
| D27⁵ | 3.00 | 4.79 | 0.080 | **<0.001** | | |
| Mean | 3.00 | 4.13 | 0.055 | **<0.001** | **<0.001** | **<0.001** |

| | | | | | | |
|---|---|---|---|---|---|---|
| ¹Least square means with their standard errors. ²The limit of detection was log₁₀ 3.00 cfu/g faeces. Values below the limit of detection were recorded as log₁₀ 3.00 cfu/g. ³SEM: standard error of the mean. ⁴Counts are from rectal swabs and are presented as Log₁₀ cfu/swab. ⁵Rectal swabs had to be taken from three pigs in the probiotic treatment group due to insufficient faecal sample. The counts from these animals are not included. | | | | | | |

Growth benefits were seen in offspring from sows fed probiotic during late gestation and lactation, which translate to carcass weight increases and hence have considerable commercial significance, as outlined above (see also Table 5 below). A unique aspect of this is that offspring were monitored all the way through to slaughter (see Table 6 below) and so it is possible to demonstrate residual benefits to pig carcass weight, while any of the EU-approved probiotics for sows only demonstrate effects during the early post-weaning period.

**Table 5: Post-weaning growth performance of piglets born to sows fed a basal diet (Control) or a basal diet supplemented with spores of the deposited strain B. altitudinis (Probiotic) from day (D) 100 of gestation until weaning (D26 of lactation)¹**

| | | Maternal treatment | | | |
|---|---|---|---|---|---|
| | Day (pw²) | Control | Probiotic | SEM³ | P-value |
| N | | 36 | 36 | | |
| Mortality⁴ | | 1 | 0 | | |
| Off trial⁵ | | 4 | 2 | | |
| | | | | | |
| Body weight (kg) | 0 | 8.5 | 8.3 | 0.33 | 0.623 |
| | 14 | 11.4 | 11.6 | 0.92 | 0.893 |
| | 28 | 18.1 | 18.7 | 0.93 | 0.616 |
| | 56 | 42.4 | 43.0 | 0.94 | 0.680 |
| | 105 | 91.7 | 95.2 | 0.98 | **0.013** |
| | 127 | 121.0 | 124.5 | 0.97 | **0.012** |
| | Mean | | | 0.42 | 0.006 |
| | | | | | |
| Average daily gain (g) | 0-14 | 214.5 | 221.0 | 17.61 | 0.795 |
| | 15-28 | 483.5 | 514.1 | 17.75 | 0.224 |
| | 29-56 | 863.9 | 868.0 | 18.45 | 0.871 |
| | 57-105 | 1024.4 | 1065.9 | 18.75 | 0.119 |
| | 106-127 | 1334.0 | 1370.0 | 18.59 | 0.173 |
| | Mean | 784.0 | 807.8 | 8.13 | **0.041** |
| | 0-127 | 890.0 | 922.3 | 10.90 | **0.042** |
| | | | | | |
| Average daily feed intake (g) | 0-14 | 292.6 | 277.3 | 29.73 | 0.716 |
| | 15-28 | 620.8 | 642.5 | 29.95 | 0.610 |
| | 29-56 | 1272.9 | 1273.7 | 30.40 | 0.985 |
| | 57-105 | 2231.4 | 2293.7 | 31.62 | 0.165 |
| | 106-127 | 3251.9 | 3322.6 | 31.36 | 0.112 |
| | Mean | 1533.9 | 1562.0 | 13.78 | 0.150 |
| | 0-127 | 1834.8 | 1883.6 | 23.75 | 0.154 |
| | | | | | |
| Feed conversion ratio | 0-14 | 1.45 | 1.28 | 0.030 | **<.0001** |
| | 15-28 | 1.30 | 1.26 | 0.030 | 0.350 |
| | 29-56 | 1.47 | 1.47 | 0.030 | 0.947 |
| | 57-105 | 2.18 | 2.16 | 0.031 | 0.682 |
| | 106-127 | 2.45 | 2.46 | 0.031 | 0.909 |
| | Mean | 1.77 | 1.72 | 0.014 | **0.019** |
| | 0-127 | 2.06 | 2.05 | 0.014 | 0.412 |

| | | | | | |
|---|---|---|---|---|---|
| ¹Least square means with their standard errors. ²pw: post-weaning. ³SEM: standard error of the mean. ⁴Mortality: Due to polyserositis and septicaemia (*Streptococcus suis* infection). ⁵Off trial: Pigs were removed from the trial due to lameness (Probiotic, N=1), pneumonia (Control, N=2), bloody diarrhoea (Control, N=1; Probiotic, N=1) and abdominal hernia (Control, N=1). | | | | | |

**Table 6: Carcass characteristics of finisher pigs slaughtered at day 127 post-weaning, born to sows fed a basal diet (Control) or a basal diet supplemented with spores of the deposited strain B. altitudinis (Probiotic) from day (D) 100 of gestation until weaning (D26 of lactation)¹**

| | Maternal treatment | | | P-value |
|---|---|---|---|---|
| | Control | Probiotic | SEM² | |
| N | 31 | 34 | | |
| Carcass weight (kg) | 90.7 | 94.8 | 1.26 | **0.029** |
| Kill out (%) | 75.1 | 75.9 | 0.20 | **0.003** |
| Lean meat (%) | 54.2 | 54.2 | 0.34 | 0.957 |
| Muscle (mm) | 48.6 | 50.6 | 1.19 | 0.230 |
| Fat (mm) | 15.6 | 16.0 | 0.42 | 0.512 |

| | | | | |
|---|---|---|---|---|
| ¹Least square means with their standard errors. ²SEM: standard error of the mean. | | | | |

The probiotic strain decreased the lactose content of the sow colostrum (P<0.01) and increased protein, true protein and casein content (P<0.05) but had no impact on milk composition (Table 7). The increased protein content of the colostrum may help to explain how the probiotic beneficially impacts offspring growth when administered to sows. It may also be indicative of increased concentrations of immunoglobulins in the colostrum of probiotic-fed sows, which would confer better immune protection to offspring, also helping to explain offspring growth benefits.

**Table 7. Colostrum and milk (day 14 post-partum) analysis of sows fed a basal diet (Control) or the basal diet supplemented with probiotic spores from the deposited strain of the claimed invention (Probiotic).**

| | **Control** | **Probiotic¹** | **SEM** | **P-value** | **Reference values²** | **Reference values³** |
|---|---|---|---|---|---|---|
| **Colostrum (%)** | | | | | | |
| Total solids | 21.97 | 24.01 | 0.581 | 0.021 | 17.2 - 30.2 | 24.8 |
| Lactose | 2.06 | 1.52 | 0.128 | 0.008 | 2.4 - 4.3 | 3.4 |
| Fat | 3.94 | 4.14 | 0.430 | 0.748 | 4.8 - 11.6 | 5.9 |
| Protein | 14.25 | 16.56 | 0.759 | 0.043 | 3.3 - 19.7 | 15.1 |
| True Protein | 13.83 | 16.18 | 0.791 | 0.047 | | |
| Casein B | 11.98 | 14.08 | 0.717 | 0.050 | | |
| | | | | | | |

| **Milk (%)** | | | | | | |
|---|---|---|---|---|---|---|
| Total solids | 18.91 | 18.56 | 0.500 | 0.627 | 18.2 - 22.0 | 18.7 |
| Lactose | 5.23 | 5.22 | 0.130 | 0.930 | 5.1 - 6.3 | 5.3 |
| Fat | 7.42 | 6.74 | 0.524 | 0.368 | 5.3 - 10.8 | 7.6 |
| Protein | 4.78 | 4.89 | 0.116 | 0.493 | 4.7 - 7.1 | 5.5 |
| True Protein | 4.25 | 4.41 | 0.115 | 0.337 | | |
| Casein B | 3.34 | 3.47 | 0.111 | 0.445 | | |

| | | | | | | |
|---|---|---|---|---|---|---|
| ¹ Probiotic: sows fed a basal diet supplemented with probiotic spores from D100 of gestation and the entire lactation period. ²Adapted from Hurley et al., 2015. Composition of sow colostrum and milk. In The Gestating and Lactating Sow; Farmer, C. (ed.), Wageningen Academic Publishers. ³Darragh, A. J. & Moughan, P. J. 1998. The composition of colostrum and milk. In The Lactating Sow; Verstegen, M. W. A., Moughan, P. J. & Schrama, J. W. (eds.), Wageningen Academic Publishers. | | | | | | |

The probiotic also favourably modulated the intestinal microbiome of the sows and/or offspring.

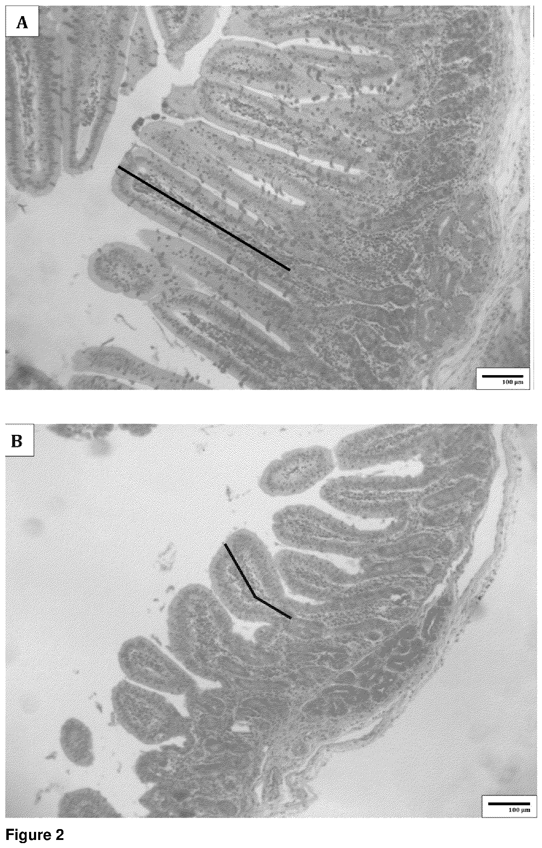

Villous height and area were also increased in the duodenum of the offspring from probiotic-supplemented vs control sows (P = 0.002 and 0.001, respectively) at day 7-8 pw and a tendency for increased villous height was observed in the ileum (P = 0.07) (see Table 8 and Figure 2). These findings indicate increased absorptive capacity in the small intestine early post-weaning, which may also help to explain the growth benefits in the offspring of probiotic-treated sows.

**Table 8: Small intestinal morphology of piglets euthanized on D7-D8 post-weaning, born to sows fed a basal diet (Control) or a basal diet supplemented with spores of the deposited strain of the claimed invention (Probiotic) from day (D) 100 of gestation until weaning (D26 of lactation)¹**

| | Maternal Treatment | | | |
|---|---|---|---|---|
| | Control | Probiotic | SEM² | P-value |
| N | 20 | 20 | | |

| **Duodenum** | | | | |
|---|---|---|---|---|
| Goblet cells (number) | 13.8 | 14.9 | 1.10 | 0.503 |
| Villous height (µm) | 352 | 393 | 8.2 | **0.002** |
| Crypt depth(µm) | 175 | 192 | 4 | **0.006** |
| VH:CD³ ratio | 2.07 | 2.09 | 0.064 | 0.869 |
| Villous area (µm²) | 40584 | 49267 | 1759.5 | **0.001** |
| Crypt area (µm²) | 6650 | 7574 | 246 | **0.013** |
| | | | | |

| **Jejunum** | | | | |
|---|---|---|---|---|
| Goblet cells (number) | 8.7 | 10.2 | 0.85 | 0.199 |
| Villous height (µm) | 347 | 362 | 8.1 | 0.189 |
| Crypt depth(µm) | 176 | 189 | 4.4 | **0.039** |
| VH:CD ratio | 2.04 | 1.96 | 0.060 | 0.384 |
| Villous area (µm²) | 38947 | 42105 | 1878.2 | 0.243 |
| Crypt area (µm²) | 6731 | 8075 | 334.4 | **0.008** |
| | | | | |

| **Ileum** | | | | |
|---|---|---|---|---|
| Goblet cells (number) | 13.6 | 15.9 | 1.29 | 0.217 |
| Villous height (µm) | 326 | 346 | 7.6 | ***0.070*** |
| Crypt depth(µm) | 183 | 187 | 4.1 | 0.509 |
| VH:CD ratio | 1.83 | 1.89 | 0.047 | 0.389 |
| Villous area (µm²) | 37552 | 41677 | 1730.2 | 0.101 |
| Crypt area (µm²) | 7211 | 7659 | 286.1 | 0.277 |

| | | | | |
|---|---|---|---|---|
| ¹Least square means with their standard errors. ²SEM, standard error of the mean. ³VH:CD: Villous height:crypt depth. | | | | |

What is also interesting to note in a study to evaluate the effects of spores of the deposited strain *B. altitudinis* administered during 1st and/or 2nd stage weaning on growth performance, health indicators and gut microbiota composition of pigs in comparison to an antibiotic (AB) zinc oxide (ZnO) combination is that the deposited strain was shed in the faeces of all pigs fed the strain. However, it was undetectable 7 days after administration had ceased. Body weight at D106 pw was higher in the AB+ZnO treatment group compared to the Pro/Pro group (P<0.05). The AB+ZnO treatment group also had higher ADG (P<0.05) than the Neg/Pro and Pro/Neg groups between D0 and 14 pw, and than the Neg/Neg, Pro/Neg and Pro/Pro groups between D15 and 28 pw. The ADFI for D15-28 pw was higher in the AB+ZnO group than in the Pro/Neg and Pro/Pro groups (P<0.05). For the overall period (D0-106 pw), the ADG and ADFI were higher in the AB+ZnO treatment group than in the Pro/Neg group (P<0.05). The gain:feed (G:F) for D0-14 pw was lower in the Pro/Neg group than in the Pro/Pro and AB+ZnO groups (P<0.05), with the highest G:F found in the AB+ZnO group (P<0.05).. None of the treatments impacted faecal scores post-weaning or carcass characteristics at slaughter. Some small effects of the probiotic treatments were observed for some of the hematological parameters measured; however, all values were within the normal ranges reported for pigs of the same age. Hence, it was assumed that these results were not of biological significance. 16S rRNA gene sequence-based profiling of the gut microbiome indicated that dietary inclusion of AB + ZnO had the greatest impact on gut bacterial populations, with large shifts occurring for some bacterial taxa, although the composition appeared to return to baseline within a few weeks of ceasing treatment. Inclusion of the probiotic spores appears to have had a more subtle impact on the intestinal microbiota composition. Overall, the results of this study show that inclusion of spores of the deposited strain of the claimed invention in the diet during 1^{st} and/or 2nd stage weaning did not affect piglet growth during the post-weaning or finisher periods (D0-106 pw).

In conclusion, this study indicated that the deposited strain of the claimed invention survived well in the GI tract of pigs, even in piglets not directly supplemented with the probiotic themselves, but whose mothers were administered probiotic. The faecal probiotic count in the piglets indicates that the probiotic had transferred successfully from the sow to offspring. In weaned pigs, there was an improvement in FCR during the period from day 0 to 14 pw in piglets from the sows supplemented with probiotic indicating improved gut health. Histological data backs this up, indicating increased absorptive capacity in the small intestine at day 7-8 pw (see Table 8 and Figure 2). The residual effect of probiotic supplementation to sows was observed at day 105 pw and day 127 pw where heavier live weights were observed. Moreover, carcass weight and kill out percentages were higher in pigs from sows supplemented with probiotics. Thus, probiotic administration to sows during late gestation and lactation, rather than direct administration to the piglets themselves, is beneficial to offspring. Considering all these, supplementation of probiotic spores to the sows is a cost-effective means of probiotic delivery to piglets to ensure early life colonization, improve gut health at the critical time of weaning and ultimately to increase pig carcass weight at their target slaughter age.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. An isolated *Bacillus altitudinis* strain as deposited with the National Collection of Industrial and Marine Bacteria under the Accession No. NCIMB 43558 on 27/01/2020.

2. A cell extract or supernatant of an isolated *Bacillus altitudinis* strain as deposited with the National Collection of Industrial and Marine Bacteria under the Accession No. NCIMB 43558 on 27/01/2020.

3. A composition comprising the isolated *Bacillus altitudinis* strain of Claim 1 or the cell extract or supernatant of the isolated *Bacillus altitudinis* strain of Claim 2.

4. The composition of Claim 3, wherein the composition is a food product, a beverage product, or a nutritional supplement.

5. The composition of any one of Claims 3 or 4, wherein the composition comprises a probiotic material and optionally a prebiotic material.

6. The composition of any one of Claims 3 to 5, wherein the isolated *Bacillus altitudinis* strain is viable or non-viable.

7. The composition of any one of Claims 3 to 6, wherein the composition comprises at least 10⁶ cfu or spores of the isolated *Bacillus altitudinis* strain per ml of composition.

8. A pharmaceutical composition comprising the isolated *Bacillus altitudinis* strain of Claim 1 or the cell extract or supernatant of the isolated *Bacillus altitudinis* strain of Claim 2, and a suitable pharmaceutical excipient.

9. The pharmaceutical composition of Claim 8, wherein the composition is provided in a unit dose form suitable for oral administration, topical administration or environmental administration.

10. A pharmaceutical composition according to any one of Claims 3 to 9 for use in a method of increasing weight gain in offspring of a monogastric animal fed or exposed to the composition.

11. A method of increasing weight gain in an offspring of a lactating mammal, the method comprising providing the lactating mammal with the composition of any one of Claims 3 to 10 during late gestation and during the lactating period.

12. The method of Claim 11, wherein the mammal is fed the composition, or the composition is applied to the body of the lactating mammal, or the composition is dispersed into the environment of the lactating mammal for about the final 14 days of gestation and for about the first 28 days of the lactating period.

13. A method of increasing weight gain in an offspring of a monogastric animal, the method comprising feeding the monogastric animal with the composition of any one of Claims 3 to 10 during late gestation and for about the first 28 days following birth.

14. The method of Claim 13, wherein the composition is applied to the body, or the composition is dispersed into the environment of the monogastric animal for about the final 14 days of gestation and for about the first 28 days following birth.

15. A method of increasing weight gain in an offspring of a bird, the method comprising injecting the composition of any one of Claims 3 to 10 *in ovo* prior to hatching.
